# EUROPEAN PATENT APPLICATION

(11) **EP 4 193 911 A1**
(43) Date of publication of application: **14.06.2023**
(21) Application number: 22210245.1
(22) Date of filing: 29.11.2022
(51) Int. Cl.: A61B 5/00, A61B 5/11

(54) **METHOD AND SYSTEM FOR GAIT EVALUATION**

(30) Priority: 30.11.2021 SE 2103033
(71) Applicant: Tequestrian AB, 703 62 Orebro (SE)
(72) Inventor: Olsson, Fredrik, 70217 Örebro (SE); Olsson, Liv, 70217 Örebro (SE); Olsson, Åsa, 70217 Örebro (SE)
(74) Representative: Olsson, Carl Fredrik

(57) **Abstract**

The invention relates to a method for determining variations in gaits for a horse wherein the method comprises the following method steps; i.) arranged sensor means on a horse, ii.) measure inertial data with the sensor means while exercising the horse, iii.) communicate measured inertial data to a handheld device, iv.) store measured inertial data on the handheld device, v.) compare currently measured inertial data with previous measured inertial data, vi.) calculate a deviation relation between the measured inertial data and the previous measured inertial data. The invention further relates to a computer program, handheld computer, a sensor, and a tendon boot and a system for determining variations in gait for a horse.

## Description

### TECHNICAL FIELD

The invention relates to a method for determining variations in gaits for a horse. The invention further relates to a computer program, handheld computer, a sensor, and a tendon boot and a system for determining variations in gaits for a horse.

### BACKGROUND OF THE INVENTION, PROBLEM AND PRIOR ART

Four-legged animals (quadrupeds), normally have a unique and healthy way of moving or ambulating (referred to as its "gait"). The gait of a quadruped is generally recognizable and consistent within a species by analysing several biomechanical signals when the animal is moving. Highly skilled and trained veterinarians are typically used to determine, by visual inspection, if an animal has an unhealthy gait caused by injury, referred to as 'lame'. However, detection of lameness in quadrupeds, namely horses and more specifically sport horses, is a significant and often subjective task for veterinarians (vets) and horse owners. Since the detection is subjective different veterinarians and horse owners commonly come to different conclusions with relation to lameness. Current methods used in clinical practice for detecting lameness in a horse are subjective and requires a highly skilled veterinarian (vet). Studies have shown that even between vets there is a certain amount of subjectivity and question about the common practices involved in detecting lameness and performing a lameness exam. The studies have shown that each vet has their own way of detection and examination. Subjectivity and lack of common practice has led owners to seek multiple vet opinions for examination before acting. This delay and subjectivity in detecting an injury in a sport horse will delay treatment, cost more, and possibly hinder the horse's future healing and success.

A veterinary exam and lameness workup is currently the most common technique for evaluating lameness in horses. A lameness workup typically includes the vet watching the horse move at a walk and a trot (symmetrical gaits) across hard and soft surfaces, in a straight line and in a circle. The symmetry of the trot allows the vet to try and visually determine asymmetry of movement in the horse to pinpoint lameness. However, such diagnosis is prone to subjectivity, and when the lameness is subtle, studies have shown disagreement between vets.

Automation is a recent area of research regarding lameness diagnostics. The goal of automation is to seek a more objective examination. The most common automation method used involves attaching many sensors (ranging from just a few to a hundred) to the horse, then filming the horse trotting with high-speed cameras and tracking the sensor's movement. Currently the most accurate digital detection apparatus for automated lameness evaluation involves the animals trotting across force plates to measure how they distribute their weight amongst limbs. Force plates are weight measuring plates embedded in the ground which can digitally detect the weight of each point of contact on them. If a quadruped can be shown to not be putting more weight on one side leg than the other, lameness can be detected. However, both methods require expensive custom setups which require the horse to be brought to the diagnosis facility and are incapable of remotely diagnosing a horse. They also require placing a harness and special sensors on the horse making it a less natural movement. Other methods of diagnosing lameness include attaching accelerometers on the horse and measuring difference in acceleration of different parts of the horse's body. Finally, custom horseshoes that can detect weight have been proposed as well, but all attempts have been too expensive and been deemed too time intensive to re-shoe the horse any time a lameness examination is required.

To detect an injury or lameness in a quadruped, the animal must be moving. No research has shown that injury to a leg can be shown while an animal is standing, while extensive research has shown differences in the ways quadrupeds walk and trot. Often pain will only occur due to movement and quadrupeds will compensate for this by changing how weight is distributed between their legs during these acts, compensating for the distribution of weight with their other body parts such as a head bob (a more pronounced upward movement of the quadruped's head during movement of a lame leg), changing the stride length, and changing the frequency and timing of the movement by individual legs.

Lameness can present itself in very subtle ways and thus precise measurements of the relative body parts (legs, head, haunches, among others) is essential. Greater accuracy can yield early detection and different degrees of lameness.

It is important to conduct detection of lameness when a quadruped moving naturally. Quadrupeds and especially sport horses have a unique speed of each of their gaits which can vary between animals. For example, two different sport horses may begin to trot at different forward speeds depending on many factors. Treadmills are often used as part of lameness detection because a horse can be forced to be in one place for an extended period time. However, it is impossible to make a treadmill that exactly match a quadruped's natural speed for their gait. As a result, the quadruped may make changes to its gait which can interfere with automated lameness detection. Force plates can allow for detecting natural movement, but they suffer from the fact that only a few strides can be detected because of the long stride length of some quadrupeds.

All methods of lameness detection, both manual and automated, aim to be non-invasive. Invasive lameness detection methods, such as X-Rays and MRI's require sedation of an animal, which is often a difficult and expensive procedure, and the horse needs to be transported to a clinic where the examination is conducted. Further, anaesthesia puts the health of the animal at risk. The animal's weight is also a factor, as it is difficult to get an unconscious and heavy animal into an MRI and there is a risk that the horse is injured by its own weight.

Current methods of automated lameness detection that require an expensive or time intensive setup and thus are not suited for regular use over an animal's lifetime and are only used at times when the quadruped is unhealthy. A less expensive system that does not require extensive setup on a horse could be used when a quadruped is healthy as well is of interest to improve the horse general wellbeing. Typically, a sport animal is only brought to see a trained veterinarian when an owner notices something is felt by a rider or visible to their eye in the movement of a quadruped. A system that is used regularly could pick up changes in the gait of the animal imperceptible to a typical owner and alert a veterinarian at an earlier stage. It would have the additional benefit of being able to identify healthy traits in an individual animal, such as sidedness of horses that can cause misdiagnosis of lameness.

For the forgoing reasons, there is a need for an automated system which can noninvasively examine a quadruped in motion using accurate measurements taken from the natural movements to timely diagnose and then, potentially, treat chronic and acute injuries.

### OBJECT AND FEATURES OF THE INVENTION

The present invention is directed to a system and method that satisfies this need. The system comprises an apparatus which can timely, accurately and automatically measure data from a quadruped while in natural motion during riding when the horse is excercised.

The system is intended to measure the horses over time, even at times when they are healthy. The system measures a baseline that will further help with the lameness diagnosis and rehabilitation. This is an improvement over existing automated lameness detection systems, as typically veterinarians only examine these horses when they are already lame. Compilations of repeated observations of individual animals, and the posture or altering posture thereof, over extended periods of time (longitudinal studies) of the quadruped movement, may allow earlier and more fine-tuned diagnosis of problems. It may also be used for rehabilitation to quantify when the horse has returned to its healthy movement. Preferably a score, or other indication, is utilized to indicate when the gait of the horse is normal or when there is indication of lameness.

The present invention relates to a method for determining variations in gaits for a horse wherein the method is performed in a computer device using sensor data from sensor means and comprises the following method steps;
i.) arrange sensor means on a horse limb,
ii.) measure inertial data with the sensor means while exercising the horse,
iii.) communicate measured inertial data to a handheld device,
iv.) store measured inertial data on the handheld device,
v.) compare currently measured inertial data with previous measured inertial data on the handheld device,
vi.) calculate a deviation relation on the handheld device between the measured inertial data and the previous measured inertial data.

According to further aspects of the improved method for determining variations in gaits for a horse;
**the** sensor means for measure inertial data comprises at least one motion sensor, rotation sensor, magnetometer sensor and/or barometric altimeter sensor.
**the** sensor means are one accelerometer arranged to a wireless communication device arranged on the left forelimb and one accelerometer arranged to a wireless communication device arranged on the right forelimb.
**the** sensor means comprises the sensors arranged in the handheld device.
**the** handheld device is arranged to the saddle of the horse.
**the** deviation is calculated by identifying a repetitive pattern in the sensor data and classifying the repetitive pattern to be either; walk, trot, canter, gallop or ambling gate and comparing the identified repetitive pattern to a previous stored pattern for the specific class of either walk, trot, canter, gallop or ambling gate.
**the** calculated deviation relation is presented on the screen of the handheld device in the form of a percentual congruency of the measured inertial data and the stores measured data such that a 100 % congruency means that the measured data and the stored measured data are identical and thus that there is no deviation in gait.

The invention further relates to a computer program for determining variation in gaits for a horse comprising program code, which, when said program code is executed on a computer performs the method steps according to any of the above.

The invention further relates to a handheld computer device comprising at least one microprocessor, at least one wireless receiver and at least one application for executing a computer program according to above for determining variation in gaits for a horse or an/or an application for communicating information to a server and/or an externally arranged computer device executing the computer program according to above for determining variation in gaits for a horse.

The invention further relates to sensor means wherein the sensor means comprises an accelerometer arranged to a wireless communication device and wherein the sensor means are adapted to be arranged to a horse and where the sensor means are arranged to;
i.) measure inertial data with the sensor means while exercising the horse,
ii.) communicate measured inertial data to a handheld device.

The invention further relates to a tendon boot arranged with a pocket for keeping sensor means.

The invention further relates to a system for determining variations in gait for a horse characterized by that the system comprises a handheld computer according to above and at least one sensor means according to above arranged wherein the sensor is arranged in a tendon boot according to above.

### ADVANTAGES AND EFFECTS OF THE INVENTION

The disclosed method describes how to keep track on the gait, and potential variation of gait, of a sport horse during normal exercise/use of the horse by utilizing a conventional mobile phone, i.e. smart phone, as a handheld device and at least one sensor to measure the movement of at least one limb of the horse.

### BRIEF DESCRIPTION OF FIGURES

Different embodiments of the invention are described in more detail below with reference to the attached figures, in which:
Fig. 1 shows the method steps of determining variations in gait according to an embodiment of the invention.
Fig. 2 shows a system realization for the method of determining variations in gait according to an embodiment of the invention.
Fig. 3 shows placement of a sensor on a horse according to an embodiment of the invention.
Fig. 4 show point of interests in a detailed image of the hoof according to an embodiment of the invention.
Fig. 5 shows point of interests on a horse according to an embodiment of the invention.

### INTRODUCTION TO DESCRIPTION

In the Summary above and in this Detailed Description, and the claims below, and in the accompanying drawings, reference is made to particular features (including method steps) of the invention. It is to be understood that the disclosure of the invention in this specification includes all possible combinations of such particular features. For example, where a particular feature is disclosed in the context of a particular aspect or embodiment of the invention, or a particular claim, that feature can also be used, to the extent possible, in combination with and/or in the context of other particular aspects and embodiments of the invention, and in the invention generally.

The term "comprises" and grammatical equivalents thereof are used herein to mean that other components, ingredients, steps, among others, are optionally present. For example, an article "comprising" (or "which comprises") components A, B and C can consist of (i.e., contain only) components A, B and C, or can contain not only components A, B, and C but also contain one or more other components.

Where reference is made herein to a method comprising two or more defined steps, the defined steps can be carried out in any order or simultaneously (except where the context excludes that possibility), and the method can include one or more other steps which are carried out before any of the defined steps, between two of the defined steps, or after all the defined steps (except where the context excludes that possibility).

The term "at least" followed by a number is used herein to denote the start of a range beginning with that number (which may be a range having an upper limit or no upper limit, depending on the variable being defined). For example, "at least 1" means 1 or more than 1. The term "at most" followed by a number (which may be a range having 1 or 0 as its lower limit, or a range having no lower limit, depending upon the variable being defined). For example, "at most 4" means 4 or less than 4, and "at most 40%" means 40% or less than 40%. When, in this specification, a range is given as "(a first number) to (a second number)" or "(a first number)-(a second number)," this means a range whose limit is the second number. For example, 25 to 100 mm means a range whose lower limit is 25 mm and upper limit is 100 mm.

### DEFINITIONS

Accelerometer: An accelerometer is a tool that measures proper acceleration. Proper acceleration is the acceleration (the rate of change of velocity) of a body in its own instantaneous rest frame.

Barometric altimeter: An altimeter or an altitude meter or a barometric altimeter is an instrument used to measure the altitude of an object above a fixed level.

Gait: The way, or manner, an animal, such as a quadruped, walks or runs.

Gyroscope: A gyroscope is a device used for measuring or maintaining orientation and angular velocity.

INS: An inertial navigation system (INS) is a navigation device that uses a computer, motion sensors (accelerometers) and/or rotation sensors (gyroscopes) to continuously calculate by dead reckoning the position, the orientation, and the velocity (direction and speed of movement) of a moving object without the need for external references. The inertial sensors could optionally be supplemented by a barometric altimeter and/or by magnetic sensors (magnetometers) and/or speed measuring devices. In this application INS is used to describe the system to determine the positions, orientation, velocity and acceleration. The INS comprises at least one motion sensor, rotation sensor, magnetometer and/or barometric altimeter.

IMU: An inertial measurement unit (IMU) is an electronic device that measures and reports a body's specific force, angular rate, and sometimes the orientation of the body, using a combination of accelerometers, gyroscopes, and sometimes magnetometers. In this application IMU is used to describe the sensors used by the INS.

Lameness: An abnormal gait or stance of an animal that is the result of dysfunction of the locomotor system, typically caused by injury but also of overwork or after extensive riding.

Locomotor: The muscular and skeletal system of the animal which governs movement.

Longitudinal Studies: Repeated studies of the same points over time, in order to establish a baseline and changes from that baseline over time.

Machine Learning: A technique of computer science where a computer is taught to recognize, or act based of a set of learned examples instead of being explicitly programmed.

Magnetometer: A magnetometer is a device that measures magnetic field or magnetic dipole moment. Some magnetometers measure the direction, strength, or relative change of a magnetic field at a particular location.

Mathematical representation of movement: Part of the processed data, indicating the positions, velocities, and accelerations of all points of interest as well as center of mass of a quadruped at a plurality of points in time.

MEMS: Microelectromechanical systems (MEMS), also written as micro-electromechanical systems (or microelectronic and microelectromechanical systems) and the related micro-mechatronics and microsystems constitute the technology of microscopic devices, particularly those with moving parts.

Motion sensor: A sensor used to measure the motion of an object, commonly by measuring the change of velocity and/or acceleration of an object.

Point Cloud: A series of points representing the position of objects in space. These points are usually defined by X, Y, Z coordinates, and often are intended to represent the external surface of an object. This is an example of a 3-Dimensional Representation.

Point of Interest (POI): A point on the body of a quadruped calculated and tracked over time.

Quadruped: An animal or species of animal having four legs and feet or hooves.

Rotation sensor: Rotation Sensors are a type of sensor used for measuring rotary displacement, rotary orientation and/or angular velocity, commonly by utilizing a gyroscope or equivalent sensor.

Session: A period of time in which a specimen quadruped is exercised.

Specimen: An individual animal or quadruped.

Symmetric Gait: A gait in which the left and right legs alternate equal movement. Humans do this naturally. With horses the only symmetric gaits are the walk and the trot, all other gaits are asymmetric.

### DETAILED DESCRIPTION OF EMBODIMENTS

An inertial navigation system, INS, is a self-contained device consisting of an inertial measurement unit, IMU, and computational unit. The IMU is typically made up of a 3-axis accelerometer, a 3-axis gyroscope and, optionally, a 3-axis magnetometer. The IMU measures the angular rate and acceleration of the system arranged with an IMU. The computational unit, arranged to the IMU, determine the attitude, position, and velocity of the system based on the raw measurements from the IMU given an initial starting position and attitude where attitude comprises angles of rotation in three dimensions about the device center of gravity, known as pitch, roll and yaw.

An inertial navigation system, INS, uses an inertial measurement unit, IMU, comprising accelerometers, gyroscopes, and sometimes magnetometers. The gyroscope angular rate measurements are integrated for a high-update rate attitude solution, while the magnetometer (if used) provides a heading reference similar to a magnetic compass.

Commonly the accelerometer, gyroscope and magnetometer could be arranged in one single integrated circuit commonly utilizing MEMS-technology.

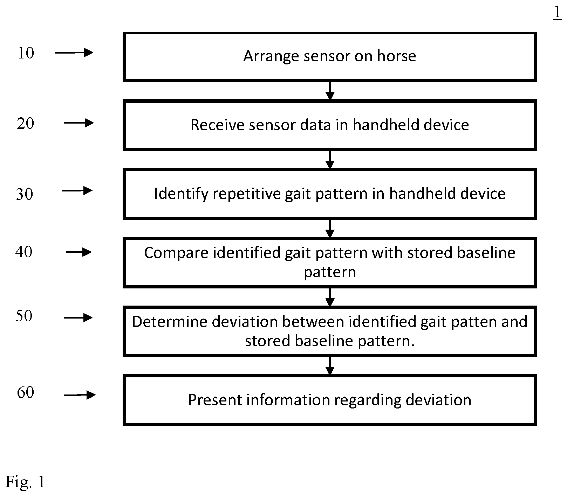

Fig. 1 shows block diagram of the steps of the method for determining deviation in gait 1. The first step of the method is to arrange sensors to a horse 10. Preferably sensors are arranged on the left and/or right forelimb, additionally a sensor could be arranged on the left and/or right hindlimb, additionally a sensor could be arranged on the head of the horse. Additionally, the sensors arranged in a handheld device, i.e. a mobile device such as a smart phone, arranged on the user (i.e. a rider) of the method where the handheld device could be arranged in a pocket on the rider or arranged to the saddle of the horse. In the next step sensor data from the sensors are transmitted to the handheld device, i.e. the handheld device receives information in step, Receive sensor data in handheld device 20. In the next step, identify repetitive gait pattern in handheld device 30, repetitive patterns of the gait are identified. Since every type of gait comprises a repetitive movement of the limbs it is possible to identify a repetitive pattern. The fourth step, compare identified gait pattern with stored baseline pattern 40, a comparison between the identified gait pattern and a previously stored baseline gait pattern is performed. The comparison could include a stored mathematical representation of movement and the new identified mathematical representation of movement or the stored point cloud and the new identified point cloud. The identified gait pattern and the stored baseline gait pattern are compared in the fifth step, determine deviation between identified gait patten and stored baseline pattern 50. If the identified gait pattern and the stored baseline pattern are determined to be identical then there is no deviation between the identified gait pattern and the stored baseline pattern. If there is a deviation between the stored baseline pattern and the identified gait pattern the deviation could be presented to the user. In the sixth step, Present information regarding deviation 60, the deviation is presented to the user on the handheld device. Deviation could be presented as a metric in deviation, as an example as a percentage of correlation between the identified gait pattern and the stored baseline pattern wherein 100 % means identical patterns with a decrease in percentage depending upon the deviation. Deviation could also be presented in the form of information. Example of information could include, as an example, a possible injury, such as suspected lameness at a specific limb of the horse. A baseline gate is determined by capturing the gate in the initial usage of the system on an animal. The baseline gate is stored in the handheld device and could also be uploaded and stored in an external service, such as a cloud storage. When using the system, the baseline gait data for the individual animal is loaded for comparison with the new gait information and a determination is made if there is a deviation that meets the programmed threshold from the baseline is made. A determination if there is a deviation between lateral sides of the quadruped is made that meets the programmed threshold from the baseline. By using this system to monitor the particular sport horse continuously for even slight deviations from the baseline, the system detects even subtle deviations in gait, or other posture or movement characteristic detailed in the processed data, to alert caretakers before any potential injury becomes exacerbated by continued exertion and/or lack of attention and treatment. It is possible to configuring thresholds for changes in lameness signals by altering parameters in the application. The baseline gait is preferably stored when the horse is believed to be healthy, the baseline gait could be updated if it is believed that the horse has reached a new level of training or for other reasons. It is also possible to aggregate several sessions with the horse to provide a baseline gait comprising the gait from several individual sessions. The method for gait evaluation is implemented in software preferably arranged in a handheld device, such as a mobile phone, i.e. a smart phone, application. Part of the system, or the complete system, could also be arranged in a remote service provider such as a server or in the cloud. The software application comprises a data set with information such as;
- animal name/identification, which is a unique reference to a specimen that is being examined,
- reference to historical data, which is a means to look up previous data for the specimen or the specimen's species,
- session Length, the length in time of a session measured in seconds,
- maximum, minimum, average, and standard deviation of each hoof contact period as measured in seconds,
- maximum, minimum, average, and standard deviation of each fetlock angle when each foot is at rest during the session measured in degrees,
- maximum, minimum, average, and standard deviation of each stride period measured in seconds,
- position of the sensors,
- average maximum and standard deviation of maximum joint velocity and acceleration for each identified POI in each direction (x, y, and z), measured in meters per second,
- maximum, minimum, average, and standard deviation of each hoof contact angle measured in degrees,
- maximum, minimum, average, and standard deviation of each hoof lift off angle,
- maximum, minimum, average, and standard deviation of the center of mass movement in each direction,
- maximum, minimum, average, and standard deviation of quadrant center of mass movements and accelerations.

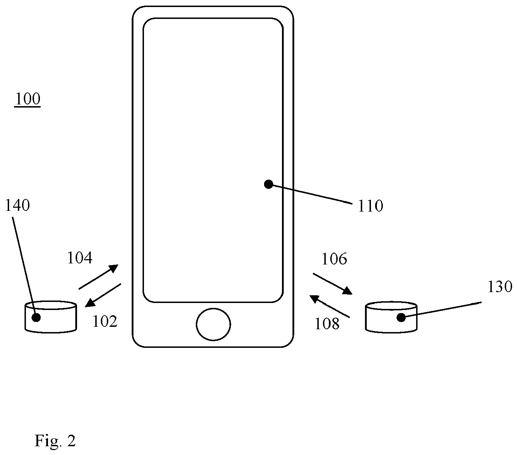

Fig. 2 shows a block diagram of a system 100 for gait evaluation comprising a handheld device 110 and two sensors 130, 140. It is possible to use the system with one sensor but two sensors are shown in this specific embodiment, it is also possible to utilize more sensors such as a sensor on every leg of the horse and additional sensors to the head of the horse and/or other positions on the horse. The sensors are arranged to transmit sensor data to the handheld device 110, indicated by arrows 104, 108. The handheld device could also optionally communicate to the sensors, indicated by arrows 102, 106. Communication to the sensors could for example be handshake information, or signals to start communications from the sensors 130, 140. The sensors are preferably an inertial sensor, such as an accelerometer and/or a gyroscope, arranged to the horse to sense the periodical movement of the horse. Streaming data from the sensors is then collected and partitioned into periods of the horse periodical movement. The data of each period is transformed into a data representation of a defined size, in order to represent each period in a normalized way thus allowing a comparison between periods. The partitioning and the transformation may be referred to as a pre-processing of time series data retrieved from the sensor. The advantage of the pre-processing is that it allows for a data representation in real-time of the periodical movement based on streaming data from an inertial sensor. Furthermore, the data representation is particularly suitable as a basis for classification of movement patterns, as well as for a calculation of different performance indicators for the movement. The data representation may thus be used for a subsequent classification of the periods into a movement class which gives as a result the class of the movement that the horse is performing during a period. Furthermore, based on the pre-processed data representation, and optionally also based on the result from the classification, a value of a performance indicator may be determined. A performance indicator is typically defined as a quantitative or qualitative measurement, or any other criterion, by which the performance, efficiency, or achievement can be assessed, often by comparison with an agreed standard or target.

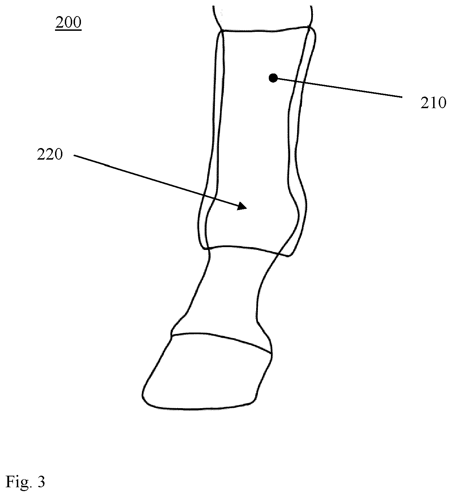

Fig 3 shows a forelimb 200 of a horse wherein a leg boot 210 is arranged to the forelimb 200, a sensor 220 is placed in or on the leg boot 210. The preferred placement of a sensor is on the left and/or right forelimb. The sensor could be arranged in a leg boot but could also be arranged in a conventional bandage. Preferable a leg boot, such as a tendon boot and/or fetlock boot, could be arranged to hold the sensor device in a pocket/holder for the sensor device.

The data from the sensors are measured over the time period of the session. For each of the sensors, arranged at a POI, a maximum acceleration and maximum velocity is be determined for each point separately. A standard deviation of that data is recorded. The positional data is then used to calculate frequency of movement (as joints accelerate and decelerate) to determine stride duration. The length of time that each hoof is in contact with the ground could be extracted from the recorded sensor data, as well as an average length of time over multiple steps as well as the standard deviation.

In some embodiments, once the system has captured and processed this data, analysis is performed on the data to determine the presence and severity of lameness (lameness signals). This analysis is done via several methods and agreement between these signals is utilized to determine certainty of lameness. The following listed methods should not be construed to be exhaustive or complete, other lameness signals known to the art could be utilized, some of the specified analysis determines that sensors are arranged on other POI on the horse but it is also possible to utilize sensors arranged on the forelimb to determine, as an example by machine learning, the specific movement patterns on the forelimbs resulting from any lameness not specifically relating to the forelimb. With an extensive data set the lameness from any POI in the horse results in a specific movement pattern on the forelimbs which could be utilized to determine the lameness only be measuring the movement pattern on the forelimbs.

One method of data analysis measures the movement in three dimensions around the hips, especially the gluteal muscles, sacrum and tuber coxae. Experience have shown that horses will elevate and drop their hip asymmetrically to compensate for pain in a hind limb lameness. The maximum, minimum, average, and standard deviation of the positions, velocities, and acceleration of dorsal points of the hip on both sides (tuber coxae covered by gluteal muscles) in all 3 coordinate planes, as well as which hooves are moving as part of the processed data. Differential in the tuber coxae movement between lateral sides of the quadruped is a lameness signal, where a greater differential indicates greater lameness.

Another method of data analysis measures head bob, which is the movement of the head in comparison with the stride of the legs. Studies' have shown that horses will utilize their heads to offset the weight that comes down on their legs during different parts of the stride. The maximum, minimum, average, and standard deviation of Y (or upward and downward) movement of the quadruped specimen's head is recorded, as well as which feet are moving and which still (stride data) form part of the processed data. Movement of the head in the Y direction is a lameness signal, where greater movement during a phase of the trot than another indicates lameness, the greater differential indicating greater lameness.

Another method of data analysis to measure the relative maximum velocities and accelerations of the hooves and, optionally, all other points of interest. It is known that a differential between the maximum velocities of the hooves appears when one leg of a horse is lame. These velocities and/or accelerations are measured in each of the X, Y, and Z dimensions for at least two legs of the horse. The maximum and minimum is recorded over the duration of a single stride. The average of each of those over multiple strides is then calculated, as well as the standard deviation. The differential between the velocity and/or acceleration between points of interest on each side of the quadruped, as well as the differential between these values and the specimen's sound baseline data, as well the differential between these values and the baseline data for the specimen species constitutes a signal of lameness, where the greater the difference indicates greater lameness.

An alternative embodiment of data analysis to determine lameness measures location where each hoof is placed in relation to the quadruped's path of movement and to the other hooves. Studies have shown that in some lameness the horse may drift away from the lame limb, so that the lamb limb tracks under the body. The location of each hoof, including maximum, minimum and standard deviation of distance of each hoof in relation to the quadruped's center of mass is used to determine lameness of an individual limb, where the greater the differential of the position of the hooves compared to the center of mass indicates lameness in that limb.

An alternative embodiment of data analysis to determine lameness measures stance, which is the symmetry in the horse as it stands still, in a resting position. Some variations in muscle mass, joint flexion, and hoof (angle, wear, shape) may vary with chronic lameness. Measurements of these will be made, including angles and shape of hoof, and all points of interest as well as joint angles will be recorded, as well as asymmetries between sides. Asymmetries in the sides is used as a lameness signal, where larger asymmetries indicate greater lameness. This data analysis is not possible to conduct with the disclosed sensor but the application in the mobile device could be arranged with a functionality to determine information from an image on the horse when the horse stands still.

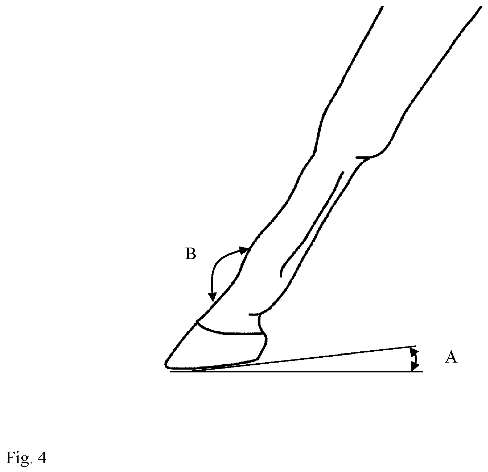

Fig. 4 shows the angle of the hoof and an alternative embodiment of data analysis to determine lameness measure of the angle of a hoof A, as shown in Fig. 4, to the ground as well as sagittal plane. This angle is measured at different points of the movement of the leg, such as at the moment when the toe is lifted off from the ground and when the hoof strikes the ground again. Forelimbs of horses have a smaller angle of their hooves to the sagittal plane (i.e. closer to the sagittal plane). If a differential exists between sides of the horse a signal of lameness is determined based on the severity of the differential. From the sensor data it is possible to determine the angle of the hoof A.

An alternative embodiment of data analysis to determine lameness utilizes the time of contact differential between the hooves on each side of the quadruped. The processed data contains a value for the average time of contact of each hoof during each step of the specimen's gait. Measuring the difference between each side of the quadruped constitutes another signal of lameness.

An alternative embodiment of data analysis to determine lameness measures the fetlock angle B, as shown in Fig. 4, of the quadruped when that leg is in contact with the ground. Studies have shown that the fetlock angle of a horse is proportional to peak vertical force that is put on an individual leg. As a result, lame legs will often have a shallower fetlock angle when in contact with the ground. The measurement of each fetlock angle at each step is part of the processed data, and the differential between fetlock angles during periods of hoof contact is a lameness signal, where a larger differential represents a larger lameness signal. By measuring the acceleration when the hoof contacts the ground it is possible to determine the fetlock angle B from the sensor data.

An alternative embodiment of data analysis to determine lameness measure differential between the normal movement of the center of gravity or center of mass for the quadruped and the measured movement. This measurement occurs in all three dimensional planes. This measurement measures the distance traveled, maximum acceleration, and maximum velocity in each direction of the center of mass.

An alternative embodiment of data analysis to determine lameness utilizes machine learning. All of the processed data for the quadruped is processed by a machine learning algorithm. This machine learning algorithm has been trained with recorded data against a plurality of horses and other quadrupeds in various states of lameness and soundness of health. The machine learning algorithm then stores with the processed data an indicator of diagnosis of both lameness and severity. The machine learning algorithms could utilize a cascading classifier, supervised machine learning algorithms such as artificial neural networks, random forest decision trees, Bayesian networks, hidden Markov models, as well as others known to the art trained on a plurality of quadrupeds in various states of lameness and soundness of health.

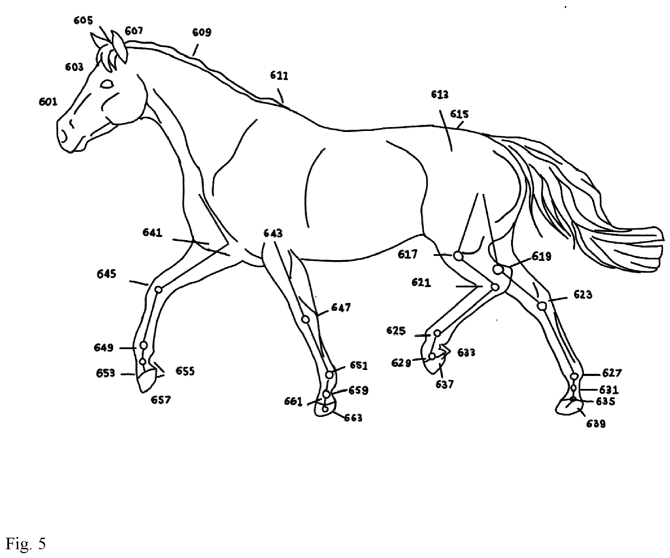

FIG. 5 shows some of the points of interest (POI) on an example quadruped. The list is illustrative to show what might be captured as a POI but should not be construed to be exhaustive or complete, other POIs could be recorded or only one POI could be recorded to make an analysis of the gait of the horse. The following POI could provide information regarding the mobility of a horse: muzzle 601, eyes 603, poll 605, ears 607, crest 609, withers 611, tuber coxae 613, sacrum 615, left stifle 617, right stifle 619, left hock 621, right hock 623, left hind fetlock 625, right hind fetlock 627, left hind interphalangeal joints 629 and 633, right hind interphalangeal joints 631 and 635, left hind limb hoof 637, right hind limb hoof 639, right forelimb elbow 641, left forelimb elbow 643, right carpus 645, left carpus 647, right forelimb fetlock 649, left forelimb fetlock 651, right forelimb interphalangeal joints 653 and 655, left forelimb interphalangeal joints 659 and 661, right forelimb hoof 657, left forelimb hoof 663.

### ALTERNATIVE EMBODIMENTS

The invention is not limited to the particular embodiments shown but can be varied in different ways within the scope of the patent claims.

The invention could be used on all quadruples.

## Claims

1. Method for determining variations in gaits for a horse **characterized by** that the method is performed in a computer device using sensor data from sensor means and comprises the following method steps;
i.) arrange sensor means on a horse limb,
ii.) measure inertial data with the sensor means while exercising the horse,
iii.) communicate measured inertial data to a handheld device,
iv.) store measured inertial data on the handheld device,
v.) compare currently measured inertial data with previous measured inertial data on the handheld device,
vi.) calculate a deviation relation on the handheld device between the measured inertial data and the previous measured inertial data.

2. Method for determining variations in gaits for a horse according to claim 1 **characterized by** that the sensor means for measure inertial data comprises at least one motion sensor, rotation sensor, magnetometer sensor and/or barometric altimeter sensor.

3. Method for determining variations in gaits for a horse according to claim 1 **characterized by** that the sensor means are one accelerometer arranged to a wireless communication device arranged on the left forelimb and one accelerometer arranged to a wireless communication device arranged on the right forelimb.

4. Method for determining variations in gaits for a horse according to any of the preceding claims **characterized by** that the sensor means comprises the sensors arranged in the handheld device.

5. Method for determining variations in gaits for a horse according to any of the preceding claims **characterized by** that the handheld device is arranged to the saddle of the horse.

6. Method for determining variations in gaits for a horse according to any of the preceding claims **characterized by** that the deviation is calculated by identifying a repetitive pattern in the sensor data and classifying the repetitive pattern to be either; walk, trot, canter, gallop or ambling gate and comparing the identified repetitive pattern to a previous stored pattern for the specific class of either walk, trot, canter, gallop or ambling gate.

7. Method for determining variations in gaits for a horse according to any of the preceding claims **characterized by** that the calculated deviation relation is presented on the screen of the handheld device in the form of a percentual congruency of the measured inertial data and the stores measured data such that a 100 % congruency means that the measured data and the stored measured data are identical and thus that there is no deviation in gait.

8. Computer program for determining variation in gaits for a horse comprising program code, which, when said program code is executed on a computer performs the method steps according to any of claims 1 to 7.

9. Handheld computer device comprising at least one microprocessor, at least one wireless receiver and at least one application for executing a computer program according to claim 8 for determining variation in gaits for a horse or an/or an application for communicating information to a server and/or an externally arranged computer device executing the computer program according to claim 8 for determining variation in gaits for a horse.

10. Sensor means **characterized by** that the sensor means comprises an accelerometer arranged to a wireless communication device and wherein the sensor means are adapted to be arranged to a horse and where the sensor means are arranged to;
i.) measure inertial data with the sensor means while exercising the horse,
ii.) communicate measured inertial data to a handheld device.

11. Tendon boot arranged with a pocket for keeping sensor means **characterized by** that the tendon boot is arranged with sensor means is according to claim 10

12. System for determining variations in gait for a horse **characterized by** that the system comprises a handheld computer according to claim 9 and at least one sensor means according to claim 10 arranged wherein the sensor is arranged in a tendon boot according to claim 11.
